(19) Europäisches Patentamt / European Patent Office / Office européen des brevets

(11) **EP 3 855 447 A1**

(12) **EUROPEAN PATENT APPLICATION**

(43) Date of publication:
28.07.2021 Bulletin 2021/30

(21) Application number: 21152971.4

(22) Date of filing: 22.01.2021

(51) Int Cl.:
*G16H 20/30* (2018.01)       *G16H 40/63* (2018.01)
*G16H 40/67* (2018.01)       *G16H 50/20* (2018.01)
*G16H 50/70* (2018.01)

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **23.01.2020   FI 20205071**

(71) Applicant: **Oura Health Oy
90590 Oulu (FI)**

(72) Inventors:
• **ERKKILÄ, Mika**
 **90590 Oulu (FI)**
• **KINNUNEN, Hannu**
 **90590 Oulu (FI)**

(74) Representative: **Berggren Oy
P.O. Box 16
Eteläinen Rautatiekatu 10A
00101 Helsinki (FI)**

(54) **METHOD FOR OPTIMIZING STRETCHING ACTIONS**

(57)   The present invention introduces a method for guiding an optimal stretching time to a user (102) who has a wearable electronic device (104) and a mobile communication device (106) for measuring a set of measurement data comprising activity data of the user (102). The determination logic, implemented e.g. in a form of a server (108), takes age and gender into account, and determines a stretching index based on the activity of the user (102). The optimal stretching time may be alerted, with stretching guidance, to the user (102). Sleep can be taken into account as well. The system measures whether the actual stretching is done. Sleep is further analyzed, so the effect of the stretching can be monitored and this information can be given back to the user (102) via the mobile communication device (106). Stretching guides can be updated as well. The wearable electronic device (104) can be a ring device.

00000

**19:55**
Tuesday, November 6

■ OURA                now

**Your optimal stretching
time is approaching.**
Your optimal stretching
time today is between
20:00 and 21:00.
Your stretch today is lower
body static stretch totally
30 minutes.

Figure 12

EP 3 855 447 A1

## Description

## TECHNICAL FIELD

The present invention relates generally to stretching optimization of a person doing fitness or physical training activity.

## BACKGROUND

Stretching is a method and activity to heal and refresh human body, and especially muscles and tendons. There are many ways to stretch and different kinds of definitions to stretching activities. Wikipedia (https://en.wikipedia.org/wiki/Stretching) teaches the following.

Stretching is a form of physical exercise in which a specific muscle or tendon (or muscle group) is deliberately flexed or stretched in order to improve the muscle's felt elasticity and achieve comfortable muscle tone. The result is a feeling of increased muscle control, flexibility, and range of motion. Stretching is also used therapeutically to relieve cramps and muscle pains.

Increasing flexibility through stretching is one of the basic tenets of physical fitness. It is common for athletes to stretch before for warming up and after exercise for reducing risk of injury and for increasing performance.

Stretching can be harmful or injurious when performed incorrectly. There are many techniques for stretching in general, but depending on which muscle group is being stretched, some techniques may be ineffective or detrimental, even to the point of causing hypermobility, instability, or permanent damage to the tendons, ligaments, and muscle fiber. The physiological nature of stretching and theories about the effect of various techniques are not well known. There are different opinions and statements for pros and cons of stretching in different situations.

For example, static stretching as a part of some warm-up routines, a study indicated that it weakened muscles. So dynamic stretching is recommended before exercise, while static stretching helps to reduce muscle soreness afterwards.

According to Wikipedia article https://en.wikipedia.org/wiki/Stretching, there are five different types of stretching: ballistic, dynamic, SMF (i.e. Self-Myofascial Release) stretching, PNF (i.e. Proprioceptive Neuromuscular Facilitation) stretching, and static stretching. Ballistic stretching is a rapid bouncing stretch in which a body part is moving with momentum that stretches the muscles to a maximum. Dynamic stretching is a walking or movement stretch. PNF is a type of stretch for a particular muscle and its specific job, so resistance should be applied, and then the muscle should be relaxed. Static stretching is a type of stretch where a person stretches the muscle until a gentle tension is felt and then holds the stretch for thirty seconds or until a muscle release is felt, without any movement or bouncing. The SMF stretching can be performed by using e.g. a tennis or a golf ball, or a foam roller as an assisting tool, and this type of stretching works by targeting soft connective tissue.

Although many people engage in stretching before or after the exercise, the medical evidence has shown that this has no meaningful benefits in preventing muscle soreness.

Stretching does not appear to reduce the risk of injury during the exercise. There is some evidence that pre-exercise stretching may increase range of movement for the athletes.

It is known that stretching will be beneficial in many cases whereas there are cases when it does not seem to give any benefits and being even harmful. The problem to a fitness exerciser is that there is no tool to tell if it makes sense to stretch and if so, when it is an optimal time to do that, and further, which kind of stretching is optimal and how much.

Patent application publication US 2011/0184247 ("Contant") discloses a health guidance system which provides suggestions to the user based on current monitored activity or length of time since a previous event, and the system can alert the user and provide routine exercise or activity suggestions for good health. Contant is however very generic and it does not guide to optimal stretching according the activity done before or follow up the stretching which has been performed and follow effects of stretching.

Patent application publication US 2017/0206795 ("Kaleal") discloses a method to provide a virtual coach for a user based on biochemical data and physiological state. The virtual coach (i.e. avatar) will generate a program and guidance for a user and follow if the user is deviating from the program. Kaleal does not discuss about optimal stretching related to previous activities or disclose any link between physical training and stretching. Also, it does not disclose optimal timing or activity-based stretching level and volume and feedback related to the performed stretching.

Thus, prior art documents present clear problems which need to be tackled.

## SUMMARY

The present invention introduces a method for providing optimal stretching guidance to a user (102) by analyzing physical activities of the user (102), in a first aspect of the present invention. The method comprises the steps of:

- collecting (1302-1602) a set of information related to the user (102) comprising an age and gender;

- measuring and receiving (1304-1604) a set of measurement data related to the user (102) comprising activity data of the user (102) by a mobile communication device (106) or by a wearable electronic device (104);

- determining (1308-1608) a stretching index for the user (102), based on the set of information related to the user (102), and the set of measurement data related to the user (102), the stretching index basing at least on the activity data of the user (102);

- providing stretching guidance to the user (102) via the mobile communication device (106), where the stretching guidance is based on the stretching index for the user (102).

The method is characterized in that the method further comprises the step of

- providing feedback to the user (102) related to the effect of the stretching done, via the mobile communication device (106).

In an embodiment of the present invention, the stretching index comprises at least one of the following: an optimal stretching type, an optimal amount or duration of stretching and an optimal stretching time of the day.

In an embodiment of the present invention, an optimal amount of stretching and an optimal stretching time of the day depend on an activity type, and activity volume or activity intensity during the last 24 hours.

In an embodiment of the present invention, the optimal stretching type depends on the activity type done.

In an embodiment of the present invention, the method further comprises the steps of:

- determining activity and sleep periods of the user (102), and

- determining the optimal stretching time depending on the time from a previous activity done or time from a previous wake-up or time to a next planned go-to-bed time.

In an embodiment of the present invention, the set of measurement data related to the user (102) is measured by a wearable electronic device (104) and transmitted by a mobile communication device (106) to a server (108) for analysis, or the measurement and transmission are both performed by a wearable electronic device (104) and a mobile communication device (106) as a combined device.

In an embodiment of the present invention, the mobile communication device (106) is a smartphone or a tablet, and the wearable electronic device (104) is a wrist device, a ring-type of a device placeable in a finger, or a chest-attachable device.

In an embodiment of the present invention, the method further comprises the step of:

- providing stretching guidance to the user (102) comprising at least one of stretching type, amount or duration of stretching, and stretching time of the day, wherein the stretching guidance is based on the stretching index for the user (102).

In an embodiment of the present invention, the method further comprises the steps of:

- collecting data from multiple users;

- sending collected data to a server (108) or to a cloud service;

- analyzing the data statistically or by machine-learning mathematical methods to find optimal stretching types, optimal amounts or durations of stretching and optimal stretching times of the day;

- updating at least one stretching guidance according to analysis results; and

- providing the updated at least one stretching guidance to the user (102), in place of the stretching guidance defined in the previous embodiment, with parameters of the previous embodiment, i.e. comprising at least one of stretching type, amount or duration of stretching, and stretching time of the day.

In an embodiment of the present invention, the method further comprises the step of:

- giving an alert to the user (102) for stretching according to optimal stretching time of the day a predetermined time period before the optimal stretching time of the day starts.

In an embodiment of the present invention, the method further comprises the steps of:

- measuring activity of the user (102) during the optimal stretching time; and

- determining if the user (102) has done stretching as guided.

In an embodiment of the present invention, the method further comprises the steps of:

- measuring and receiving a set of measurement data related to the user (102) comprising activity data of the user (102) from the mobile communication device (106) in following days and nights;

- analyzing sleep of the user (102) over the following 24-hour periods for a predetermined number of periods, when also stretching guidance is given to the user (102) during these periods; and

- analyzing an effect of the performed stretching to a sleep index or a recovery index or a readiness index of the user (102).

In an embodiment of the present invention, the method further comprises the step of:

- updating stretching guidance based on the results of analyzing the effect of the performed stretching to the user (102).

In an embodiment of the present invention, the collected set of information comprises at least one of weight, height, fitness level, main activity type, and training or sport type of the user (102).

In a second aspect of the present invention, there is presented a system for providing optimal stretching guidance to a user (102) by analyzing physical activities of the user (102), wherein the system comprises:

- a wearable electronic device (104);

- a mobile communication device (106); and

- a server (108);

- the server (108) is configured to collect (1302-1602) a set of information related to the user (102) comprising an age and gender;

- the wearable electronic device (104) is configured to measure and the server (108) is configured to receive (1304-1604) a set of measurement data related to the user (102) comprising activity data of the user (102) by a mobile communication device (106) or by a wearable electronic device (104);

- the server (108) is configured to determine (1308-1608) a stretching index for the user (102), based on the set of information related to the user (102), and the set of measurement data related to the user (102), the stretching index basing at least on the activity data of the user (102);

- the mobile communication device (106) is configured to provide stretching guidance to the user (102) via the mobile communication device (106), where the stretching guidance is based on the stretching index for the user (102).

The system is characterized in that

- the mobile communication device (106) is configured to provide feedback to the user (102) related to the effect of the stretching done.

In an embodiment of the present invention, the system further comprises the mobile communication device (106) which is configured to transmit the set of measurement data related to the user (102) to the server (108) for analysis, or the measurement and transmission are both configured to be performed by the wearable electronic device (104) and the mobile communication device (106) as a combined device.

In an embodiment of the present invention, the mobile communication device (106) is a smartphone or a tablet, and the wearable electronic device (104) is a wrist device, a ring-type of a device placeable in a finger, or a chest-attachable device.

In an embodiment of the present invention, the wearable electronic device (104) comprises at least one of the following: a heart rate sensor, a light sensor, an activity sensor, a temperature sensor, a rechargeable battery, an optional sensor, a microprocessor (MCU), a memory, an output indicator comprising a piezo and/or LED indicator, and a communication unit comprising wireless and/or Bluetooth transmission.

In an embodiment of the present invention, the mobile communication device (106) comprises at least one of the following: an input device comprising at least one of a touchpad, a touch display, a microphone, a camera and a battery; an output device comprising at least one of a display, a piezo element and a speaker; a rechargeable battery; an optional sensor comprising at least one of a light, location, GPS, and motion sensor; a microprocessor (MCU); a memory; a wireless communication unit to the wearable electronic device (104); and a wireless communication unit to a network (110).

In an embodiment of the present invention, a network (110) comprises at least one of the following: a microprocessor (MCU); a memory; an output indicator comprising a piezo and/or LED indicator; a wireless communication unit to the mobile communication device (106); and a wireless or wired communication unit to the server (108).

In an embodiment of the present invention, the server (108) comprises at least one of the following: an input device comprising at least one of a touchpad, a touch display, a microphone, a camera and a battery; an output device comprising at least one of a display, a piezo element and a speaker; a power unit; a microprocessor (MCU); a memory; a wireless or wired communication unit to the network (110); and a database.

In a third aspect of the present invention, there is presented a computer program product for providing optimal stretching guidance to a user (102) by analyzing physical activities of the user (102), wherein the computer program product comprises program code, which is executable when run in a processor. The computer program product is configured to execute the steps of:

- collecting (1302-1602) a set of information related to the user (102) comprising an age and gender;

- measuring and receiving (1304-1604) a set of measurement data related to the user (102) comprising activity data of the user (102) by a mobile communication device (106) or by a wearable electronic device (104);

- determining (1308-1608) a stretching index for the user (102), based on the set of information related to the user (102), and the set of measurement data related to the user (102), the stretching index basing at least on the activity data of the user (102);

- providing stretching guidance to the user (102) via the mobile communication device (106), where the stretching guidance is based on the stretching index for the user (102).

The computer program product is characterized in that the computer program product is further configured to execute the step of

- providing feedback to the user (102) related to the effect of the stretching done, via the mobile communication device (106).

**BRIEF DESCRIPTION OF THE DRAWINGS**

FIG. 1 is a schematic illustration of a system for providing optimal stretching guidance for a user, in accordance with an embodiment of the present invention;

FIG. 2 is an exemplary illustration of a circadian rhythm and daily schedule of the user, in accordance with an embodiment of the present invention;

FIG. 3 is a schematic illustration of a wearable electronic device of a system for providing optimal stretching guidance, in accordance with an embodiment of the present invention;

FIG. 4 is a schematic illustration of a mobile communication device of a system for providing optimal stretching guidance, in accordance with an embodiment of the present invention;

FIG. 5 is a schematic illustration of a network of the system for providing optimal stretching guidance, in accordance with an embodiment of the present invention;

FIG. 6 is a schematic illustration of a server of the system for providing optimal stretching guidance, in accordance with an embodiment of the present invention;

FIG. 7 is an illustration of exemplary measurement data of a person comprising heart rate, temperature and activity, in accordance with an embodiment of the present invention;

FIG. 8 is an illustration of exemplary measurement data of a person comprising motion data including activity or inactivity recognition, in accordance with an embodiment of the present invention;

FIG. 9 is an exemplary illustration of an optimal stretching time and input to select it (training, sleep time, go-to-bed time, wake-up time), in accordance with an embodiment of the present invention;

FIG. 10 is an illustration of exemplary stretching guidance in a data table form, in accordance with an embodiment of the present invention;

FIG. 11 is another illustration of exemplary stretching guidance in a data table form, in accordance with an embodiment of the present invention;

FIG. 12 is an illustration of exemplary stretching guidance on the display of a mobile device, in accordance with an embodiment of the present invention;

FIG. 13 is an illustration of steps of a first method for determining an optimum stretching, in accordance with an embodiment of the present invention;

FIG. 14 is an illustration of steps of a second method for determining an optimum stretching, in accordance with an embodiment of the present invention;

FIG. 15 is an illustration of steps of a third method for determining an optimum stretching, in accordance with an embodiment of the present invention;

FIG. 16 is an illustration of steps of a fourth method for determining an optimum stretching, in accordance with an embodiment of the present invention; and

FIG. 17 is an illustration of steps of a fifth method for determining an optimum stretching, in accordance with an embodiment of the present invention.

**DETAILED DESCRIPTION**

The following detailed description illustrates embodiments of the present invention and exemplary ways in which they can be implemented.

The present invention introduces a method for providing optimal stretching guidance to a user by analyzing physical activities of the user. A corresponding system and a computer program product are introduced as well.

The present invention gives a solution to the above-mentioned problems. It will collect information about the user by measuring and analyzing data and by providing instructions to stretch in a substantially optimal way.

The present invention will be a tool to collect information and feedback about work-loads and activity before stretching, about the stretching done, and status, feelings, recovery and readiness after the stretching. The data collected from a user and further, from multiple users will be used to analyze and optimize stretching methods, to guide to do correct and proper stretching activities, and movements, stretching time, and stretching amount or volume by stretching at the right time for that particular user. By volume, it is meant the stretching movements or repetitions multiplied by the time used for stretching.

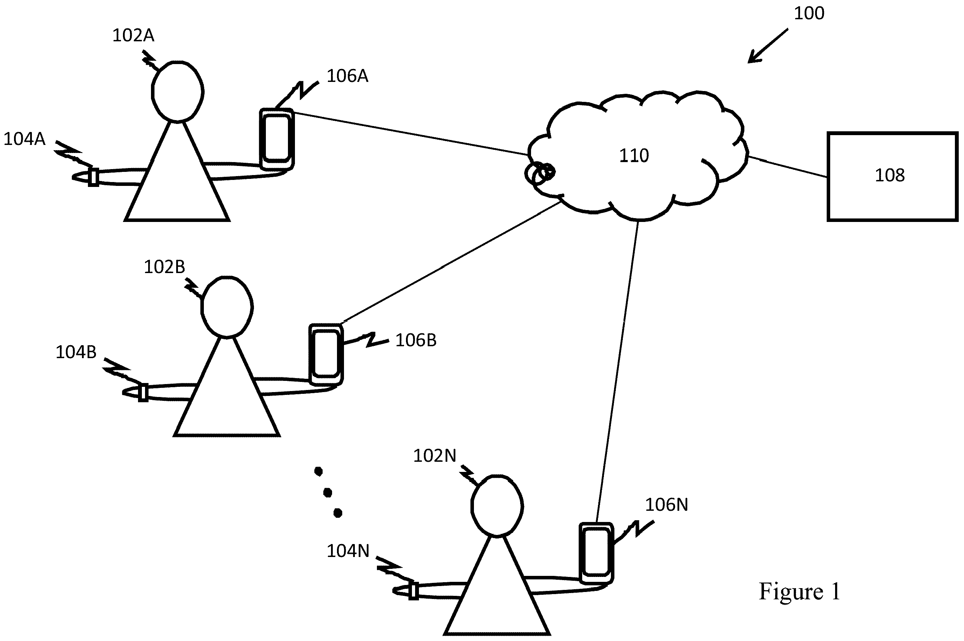

FIG. 1 illustrates an example of a general arrangement (i.e. a system) 100 according to the present invention, which enables providing substantially optimal stretching instructions (i.e. stretching guidance or scheme) to a user 102. The algorithm is based on user activity and possibly also user body temperature and heart rate measured by a wearable device 104. The "substantially" optimal means that there can be several quite good and reasonable stretching schemes for a certain user situation in a certain date, but the present invention is not necessarily restricted to select only the absolutely "best" stretching scheme. It can be also said that if the best stretching scheme for the user is "100 %" satisfactory in the situation, the present invention may select e.g. some of the schemes exceeding 90 % satisfactory levels. Thus, we have formulated the selection among the stretching instructions to be "substantially optimal", which means the same as "sufficiently optimal" considering the situation in practice.

In other words, the general system structure is illustrated in FIG. 1, showing a group of users 102 i.e. professional athletes, recreational exercisers, or in practice any desired person (meaning just a general group of different persons each locating anywhere) who are subject to the analysis according to the method according to the present invention. In other words, the main concern for the algorithm is a single user 102, but the algorithm may apply data obtained in the history from several users 102A, 102B, 102N, when performing calculations and determinations. The system or arrangement 100 comprises one or more users 102, among which users A, B and N are shown (N means any positive integer, not just fourteen users), as users 102A, 102B and 102N. Each user 102A, 102B, ..., 102N has a wearable device 104A, 104B, ..., 104N, which can be also called as a user monitoring device, which is an electronic device with at least one sensor. In an embodiment, the wearable device 104 is a wearable, smart ring. In another embodiment, the wearable device 104 is a smart wrist-held device. In yet another embodiment, the wearable device 104 comprises a sensor or a group of sensors placed on top of the human skin in a desired place, or a sensor or a group of sensors placed within a fabric of a cloth worn by the user. In this example, each user 102A-N also has a personal smartphone 106A, 106B, ..., 106N, and in a personal sense, this means that a single user 102 which is considered by the stretching instructions algorithm, has a personal smartphone 106. The smartphones 106 may provide access from the wearable smart devices 104 to the network 110; in other words, the respective smartphone 106 of the user act as gateways for the measured personal data by having a connection both to the personal wearable device 104 and to the network 110. The network 110 is represented here with a cloud symbol. As part of the network, there is a server 108 which can be a computer within the user's own premises (e.g. at home) or a computer within service provider's premises. Thus, the wearable devices 104 are all connected to the network 110, for transferring the measurement results from all desired users 102 to the server 108, and for other needed information transfer, such as personal ID information and/or other related information concerning the user(s) and their measurements.

In other words, referring still to FIG. 1, there is shown a schematic illustration of a system 100 for providing feedback to a user 102 to optimize stretching of a person doing fitness or physical training activity, in accordance with an embodiment of the present invention. The system 100 comprises a wearable electronic device 104 configured to be worn by the user 102 and a mobile communication device 106 configured to communicate with the wearable electronic device 104. The system 100 further comprises a server 108 (i.e. a server arrangement) configured to communicate with the mobile communication device(s) 106. Optionally, the server 108 is configured to communicate with the mobile communication device(s) 106 using a network 110. The server 108 and the network 110 are capable to support and communicate with multiple users 102A, 102B, ..., 102N and their mobile communication devices 106A, 106B, ..., 106N.

Optionally, the wearable electronic device 104 of the system 100 can be a ring configured to be suitably worn at a finger, such as e.g. an index finger, of the user 102. However, in an embodiment, the system 100 may be associated with other wearable electronic devices, such as a device adapted to be worn at wrist, chest or any suitable body part of the user 102, from where physiological data of the user 102 can be measured. In such an instance, the wearable electronic device 104 may be configured to have a size to be suitably worn at such a body part of the user 102.

In an embodiment, the wearable electronic device 104 comprises means for measuring a set of measurement data related to the user 102. Specifically, the set of measurement data may comprise one or more of the following: heart rate, movement of the user, temperature of the user's skin. The wearable electronic device 104 may comprise at least one sensor as means for measuring the set of measurement data related to the user 102. Furthermore, the at least one sensor may be selected from a group consisting of an accelerometer, a gyroscope and a magnetic field sensor (i.e. a magnetometer), for measuring user's movements. Furthermore, the heart rate may be measured using a photon (for example infrared, IR) source and a photon detector also arranged on an inner surface of the wearable electronic device 104. Additionally, the wearable electronic device 104 may comprise a light sensor arranged on an outer surface of the wearable electronic device 104 for measuring ambient light. A temperature sensor for measuring the temperature of the user 102 is preferably arranged against the skin of the user, for example on the inner surface side of the ring. One temperature sensor can be arranged to measure ambient temperature by arranging the sensor to be on the outer surface of the ring. The measured sensor data from the group of sensors, such as the data of the motion sensor, the optical electronics, the light sensor, the skin temperature sensor, and the ambient temperature sensor, associated with the user 102 and measured by the wearable electronic device 104 may be further analyzed to obtain the set of measurement data.

The wearable electronic device 104 comprises means for measuring a circadian rhythm and duration of sleep of the user 102. It also comprises means to measure user's activity and activity type and activity duration. Specifically, the circadian rhythm may refer to physical, mental, and behavioral changes in the user 102 that follow a daily cycle of the user. More specifically, the user 102 may experience a peak in energy levels at specific durations of time in a day. Similarly, the user 102 may also experience a drop in energy levels at specific durations of time in a day. Such changes in the user 102 may influence an overall sleep pattern thereof. Furthermore, such changes in the user 102 may be measured to estimate the circadian rhythm of the user 102.

Optionally, the duration of sleep is measured as a time between the moment of falling to sleep and the moment of waking up, wherein said moments are determined based on at least one of pre-defined changes in the heart rate and pre-defined changes in body temperature of the user 102. For example, the duration of sleep of the user may be derived from a hypnogram. Alternatively, the duration of sleep of the user may be measured with the data from the motion sensor (i.e. when the user went to bed and when the user woke up), which should be static or comprise minute variations (due to no physical provocations). Therefore, based on the data from the motion sensor, how long the user 102 slept can be determined. Furthermore, the data from the motion sensor and the hypnogram may be correlated to measure the duration of sleep.

In an embodiment, the circadian rhythm may be measured using various sensor data. Furthermore, the circadian rhythm of the user 102 may be affected by a chronotype of the user 102. As mentioned above, the wearable electronic device 104 comprises the light sensor capable of measuring illumination level as well as "colour space". The colour space refers to visible frequencies of the light. For example, if the light sensor detects blue light then the light sensor considers the light to be day light. This can be used to determine if the ambient light is from artificial light or natural light. Further, the light sensor can be used to detect illumination conditions during the sleeping time and corrected therewith. Therefore, based on the data from the light sensor, the temperature sensor and the sleeping pattern measurements, a circadian rhythm of the user can be measured. The circadian rhythm may comprise information such as at around 2 AM the user 102 gets the deepest sleep, at 4:30 AM the user 102 has the lowest body temperature, at around 6:45 AM the user 102 has the sharpest (i.e. highest) blood pressure, and so forth. These are merely exemplary times for a certain user.

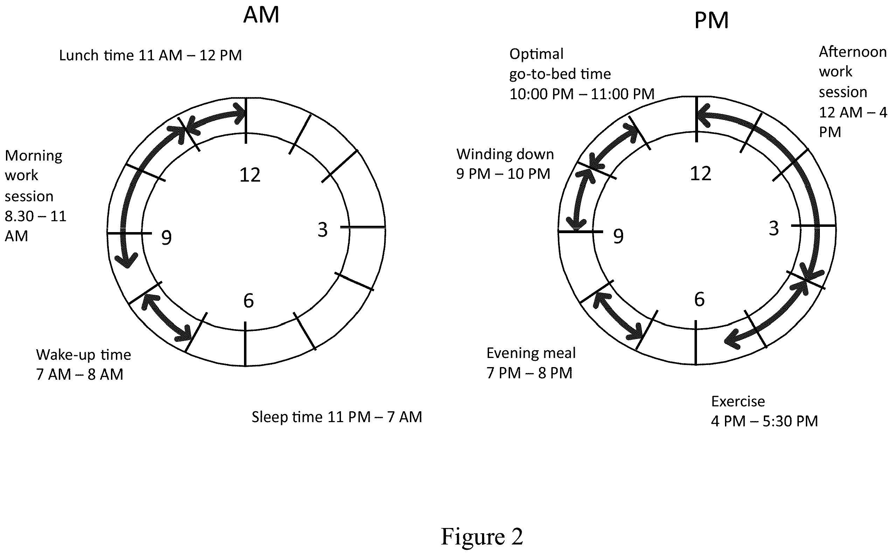

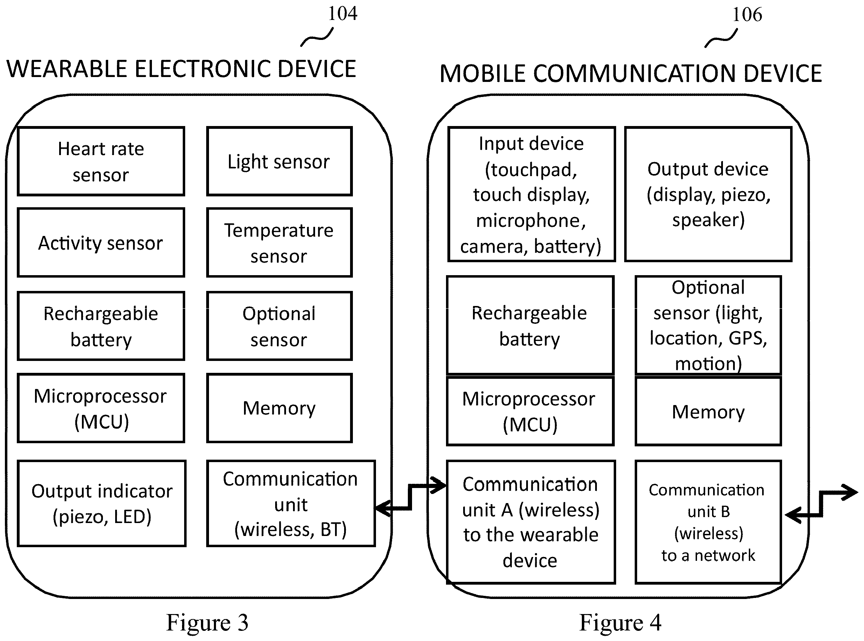

The circadian rhythm can be further extended to describe a typical day of the user 102. A typical day is described in FIG. 2, but this is of course just an example. It shows that the sleeping time of the user is 11 PM - 7 AM, wake-up time with breakfast is 7 - 8 AM, morning working session is 8.30 - 11 AM, lunch time is 11 AM - 12 PM, afternoon work session is 12 PM - 4 PM, training or exercise (when it is training time and day) is 4 - 5.30 PM, evening meal or dinner is 7 - 8 PM, and then preparing to go to bed is 9 - 10 PM and having an optimal go-to-bed time between 10- 11 PM. Days can vary and the schedule can be tuned based on the measured circadian rhythm and activities, as well as based on other information based on user's input or receiving information from the digital calendar or emails of the user. According to an embodiment, the wearable electronic device 104 also comprises electronic components configured to collect and analyze data from the at least one sensor. For example, as shown in FIG. 3, the wearable electronic device 104 may comprise other electronic components, which may comprise a controller, a microprocessor, a memory and a communication module. The controller is operable to control operation of the at least one sensor for generating data related to the user's movement, heart rate, temperatures, ambient light and ambient temperature (which the user is subjected to). The microprocessor may be operable to process or analyze collected data generated by the at least one sensor. The microprocessor can analyze one or more sensor data to recognize activity time and type of the user and inactivity time and duration of the user. Further, the memory is used for storing the analyzed or processed data. Moreover, the communication module is configured to establish communication between the wearable electronic device 104 and the mobile communication device 106. For example, the mobile communication device 106 may be wirelessly connected to the wearable electronic device 104 by a wireless connection such as a Wi-Fi, Bluetooth and so forth. Furthermore, the mobile communication device 106 is intended to be broadly interpreted to comprise any electronic device that may be used for voice and/or data communication over a wireless communication network. Examples of mobile communication devices comprise cellular phones, personal digital assistants (PDAs), handheld devices, wireless modems, laptop computers, personal computers and so forth. Additionally, the mobile communication device 106 may comprise a casing, a memory, a processor, a network interface card, a microphone, a speaker, a keypad, and a display, as shown in FIG. 4.

The mobile communication device 106 is operable to collect a set of information related to the user 102. Specifically, the first set of information may comprise information such as height, weight, age, gender, location and so forth related to the user 102. Optionally, the set of information may comprise physiological performance related information based on an external data input by the user 102. Optionally, the set of information may comprise activity habits, typical activity or sports, or typical training types and amounts, possible training plan or so. Optionally, the set of information can be automatically or semi-automatically received or grabbed by a software or application running in the mobile communication device 106. The application can for example read the electronic calendar, emails, messages etc. to find a schedule for training and activity sessions, and possibly also, which type of activity is planned and scheduled. Optionally, the set of information can comprise stretching routines or habits of the user. This may comprise, which kind of movements are familiar or already in use, and how much stretching has been done and intended to be done, at which day and/or time they have been done and how long is a single stretching session. Specifically, the user 102 may manually input information related thereto in the mobile communication device 106. Furthermore, the physiological performance related information may be derived from the physiological data (or parameters) of the user 102 measured by the wearable electronic device 104, such as heart-rate variability, a respiration rate, a sleeping pattern of the user, a hypnogram, user's stress level, activity amount and type, and so forth. Additionally, but optionally, the physiological performance related information can be biased or influenced by some external data (or factor), which is different from the internal data, such as the biological signals or physiological data associated with the user 102.

In an embodiment, the external data comprises at least one of travel information, time zone, calendar, working schedule, and holidays. The external data may be received from the user 102 as user input with the help of the mobile communication device 106. For example, the mobile communication device 106 may be provided with various user interfaces associated with such external data allowing the user 102 to make selection for the external data. Furthermore, the mobile communication device 104 may comprise sensors, such as a location sensor (e.g. for GPS) to determine the location of the user 102, i.e. if the user 102 has travelled some distance and moved out of his city/country. Further, the travel may be of such a nature which may influence sleep of the user 102. For example, this may be a travel plan which requires travelling at night, travelling to different time zones, or travelling in difficult conditions, such as in rough terrain.

Additionally, the information of the travels may be such that they may influence the physiological state (parameters or data) of the user 102, when associated with the current travel. In an example, the information of the travels may be comparatively recent (for example few days ago, or a week or a month), such that when the user takes the current travel (or a new travel), the information of the past travels and the future travels may influence the sleep of the user.

The information of travels can be taken into consideration in user's circadian rhythm and the description of the day schedule of the user 102, such as the one shown in FIG. 2. For example, if the user travel to the country which has a different time zone, this can shift the day schedule for example one hour in each day to reach the same daily rhythm related to a local time. For example, when travelling eastbound five time zones in which time is 5 hours ahead of the time of the original location, the daily schedule can be tuned earlier one hour per day during the following five days. For example, for the first day shifting the wake-up time from 7 AM to 6 AM at original location time, or 11 AM at the destination local time), the next day shifting the wake-up time to be 5 AM at original location time, or 10 AM at the destination local time. The shifting amount and duration can vary due to personal or other preferences.

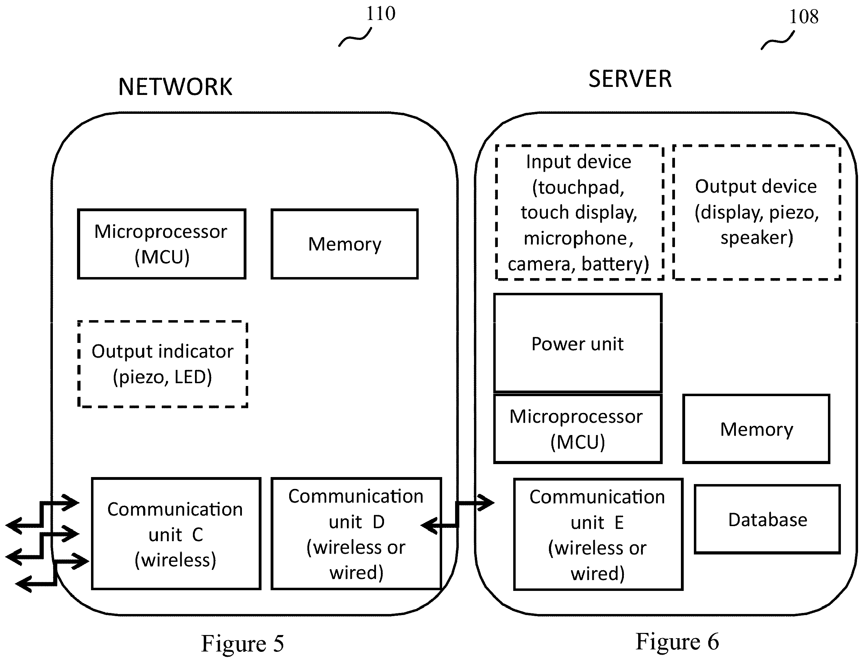

In an embodiment, the mobile communication device 106 and the server (arrangement) 108 (see FIG. 6) are configured to collect data from at least one sensor generated by the wearable electronic device 104. Furthermore, the mobile communication device 106 and the server arrangement 108 are configured to perform analysis of the data from at least one sensor in order to find heart rate variability, hypnogram, stress level, sleep duration, circadian rhythm, activity amount, activity type and activity time or the like.

Furthermore, the mobile communication device 106 and the server arrangement 108 are configured to perform analysis of the activity and inactivity data to recognize activity or inactivity periods, their duration, activity type and amount. The analysis of activity and inactivity data comprises information about activity type (for example running, walking, muscle training, Pilates or yoga, sitting, or standing or lying. Furthermore, the mobile communication device 106 and the server arrangement 108 are configured to perform further analysis of the activity and inactivity data to form a stretching index. The stretching index comprises a suitable stretching type and amount and stretching time related activity and user's circadian rhythm. The stretching time is for example optimally selected to be prior to the user's go-to-bed time or in the morning or in the afternoon.

For example, the analysis may be performed partly by the mobile communication device 106 and partly by the server arrangement 108. Alternatively, the entire analysis may be performed by the mobile communication device 106.

Throughout the present disclosure, the term "server" or "server arrangement" relates to a structure and/or a module which includes programmable and/or non-programmable components configured to store, process and/or share information. Optionally, the server arrangement 108 comprises any arrangement of physical or virtual computational entities capable of enhancing information to perform various computational tasks. Furthermore, it should be appreciated that the server arrangement 108 may be either a single hardware server or a plurality of hardware servers operating in a parallel or distributed architecture. In an example, the server may comprise components such as a memory, a processor, a network adapter and the like, to store, process and/or share information with other computing components, such as the mobile communication device 106. Optionally, the server 108 is implemented as a computer program which provides various services, such as a database service.

The server 108 block diagram is presented in FIG. 6. The server arrangement 108 is configured to communicate with the mobile communication device 106. For example, the server arrangement 108 is communicatively coupled to the mobile communication device 106 through a network 110 which can be wired, wireless or a combination thereof. The block diagram of the network 110 is presented in FIG. 5. For example, the network 110 may comprise Local Area Networks (LANs), Wide Area Networks (WANs), Metropolitan Area Networks (MANs), Wireless LANs (WLANs), Wireless WANs (WWANs), Wireless MANs (WMANs), the Internet, second generation (2G) telecommunication networks, third generation (3G) telecommunication networks, fourth generation (4G) telecommunication networks, fifth generation (5G) telecommunication networks or Worldwide Interoperability for Microwave Access (WiMAX) networks.

The server arrangement 108 is operable to receive the set of information related to the user 102 from the mobile communication device 106, and receive the set of measurement data related to the user 102, the measured circadian rhythm and the duration of sleep of the user 102 from the wearable electronic device 104 or from the mobile communication device 106. The circadian rhythm may be also defined in the server 108 based on the measurement data from the wearable electronic device 104. The server arrangement 108 is operable to determine so-called sleep scores for a predefined number of days. Specifically, the server arrangement 108 is operable to determine a sleep score for each of the predefined number of days from the first set of information, the set of measurement data, the circadian rhythm and the duration of sleep of the user 102, in an embodiment. More specifically, the server arrangement 108 may be operable to analyze the received parameters of the user 102 to determine a sleep score of a sleep of the user 102, circadian rhythm, daily schedule, activity type, activity amount, activity duration and activity time of the user 102, and stretching index of the user 102.

As the technology evolves with larger memories and faster processing capabilities in mobile communication devices, it is technically possible that the server's functions and tasks can be realized wholly or partially in a mobile communication device.

The activity of the user 102 can be measured by a motion sensor in a wearable electronic device 104. It is however possible to measure activity with a mobile communication device 106 with its motion sensor or location sensor. An activity chart can be presented, for example, as motions per minute. The sensor or measuring electronics may have a threshold for a motion signal which can be, for example, an acceleration signal of 0.05

$$0.5 \frac{m}{s^2}.$$

* g, i.e. appr. $0.5 \frac{m}{s^2}$. When the acceleration signal exceeds the threshold, a counter for motions/minute is added by one. After every minute that cumulative count is recorded and the counter is reset for counting motions for the next minute.

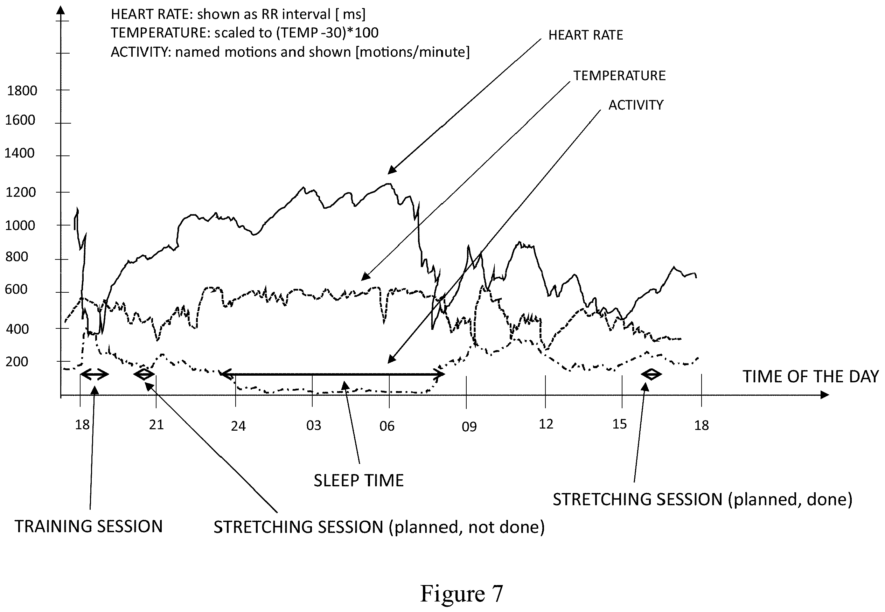

In an example, activity counts for one day are shown in a chart shown in FIG. 7. The motions per minute vary there between 0 - 400 motions/minute. The chart also shows different periods; a training session between 18-19 o'clock, sleep time between 23-07:30, and a stretching session between 16-17 o'clock next day.

Typical motions/minute values can be used as criteria to detect the activity period of the user 102. A clock time can be used to make rules to analyze the activity type. For example, during afternoon a high activity level (more than 200 motions/minute) means training; between 22-08 o'clock a low activity level (less than 20 motions/minute) means sleeping, and between 8-16 o'clock a low activity level (less than 20 motions/minute) means sitting or inactivity.

Training type can be defined from activity level, time of activity, duration of activity, and possibly from heart rate and temperature, or it can be input by a user.

Other sensor information can be used for analysis. For example, temperature is elevated during a strenuous exercise as it can be seen between 18-19 o'clock in FIG. 7. During night-time, the skin temperature may be higher, although the body core temperature may be lower. This is due to the fact that blood circulation of a finger and hand change and also because a finger or hand is typically closer to the body and may be under a blanket. The temperature curve during one day (from 17:30 to 17:30 next day) is also shown in FIG. 7. During night-time (23-7:30 o'clock), the skin temperature is elevated. The temperature also elevates during strenuous exercise (in full body exercise, such as skiing, swimming etc.) as can be seen between 18-19 o'clock.

The heart rate (HR) can be used as an indicator, too. It is known that HR responds very consistently to the amount of exercise. The chart of FIG. 7 shows the heart rate in a form of a heartbeat interval (RR) in milliseconds. 60 beats per minute thus means 1000 ms as RR interval (duration between two successive heart beats). During the exercise between 18-19 o'clock, the RR is averagely about 400 ms (corresponding to 150 beats per minute), and during the sleeping the RR is between 900-1200 ms. These values may of course differ significantly in different kinds of people.

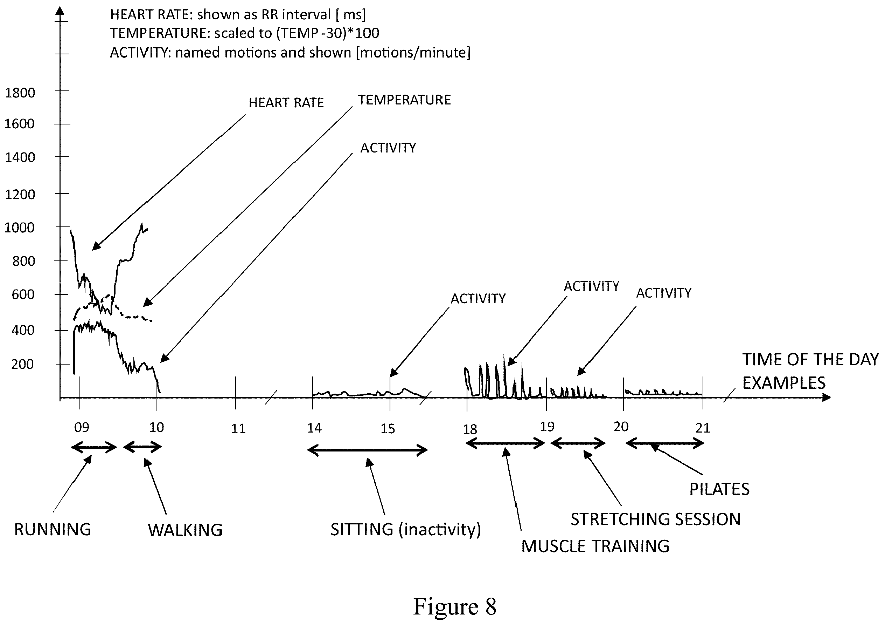

FIG. 8 shows different activities having different sensor data measured by a motion sensor or a heart rate sensor or a skin temperature sensor, in an example.

Training type can be defined from activity level, time of activity, duration of activity, and possibly from heart rate and temperature, or it can be input by a user.

Different rules can be set to analyze and differentiate different activity types.

For example, following rules can be used:

Running: activity > 300 motions/min, period 0.5-1 steps/s (optionally RR < 600 ms)

Walking: activity > 60 motions/min, but < 250 motions/min, period 0.5-1 steps/s (optionally RR < 750 ms)

Sitting: activity < 20 motions/min (during working hours)

Muscle training: activity peaks > 60 motions/min followed by activity valleys < 30 motions/min, periods repeating 30-240 s cycles

Stretching: activity peaks > 30 motions/min but < 50 motions/min followed by activity valleys < 10 motions/min, periods repeating 30-300 s cycles

Pilates or yoga: activity peaks > 20 motions/min but < 30 motions/min followed by activity valleys < 5 motions/min, periods repeating 30-300 s cycles

The rules can be tuned and personalized in different embodiments. Also advanced machine learning and artificial intelligence (AI) tools can be used to enhance the system with more accurate analysis methods.

The optimal stretching time depends on different things. At some day it might be reasonable to do the stretching at the same day as the exercise, and sometimes it is fine to do the stretching the next day after the exercise. The stretching time can be in the morning, in the afternoon or in the late evening. However, it is important to adapt the stretching time to the user's schedule so that it is fitting to the other daily routines of the user and it is not disturbing, for example, the go-to-sleep time or the sleeping itself.

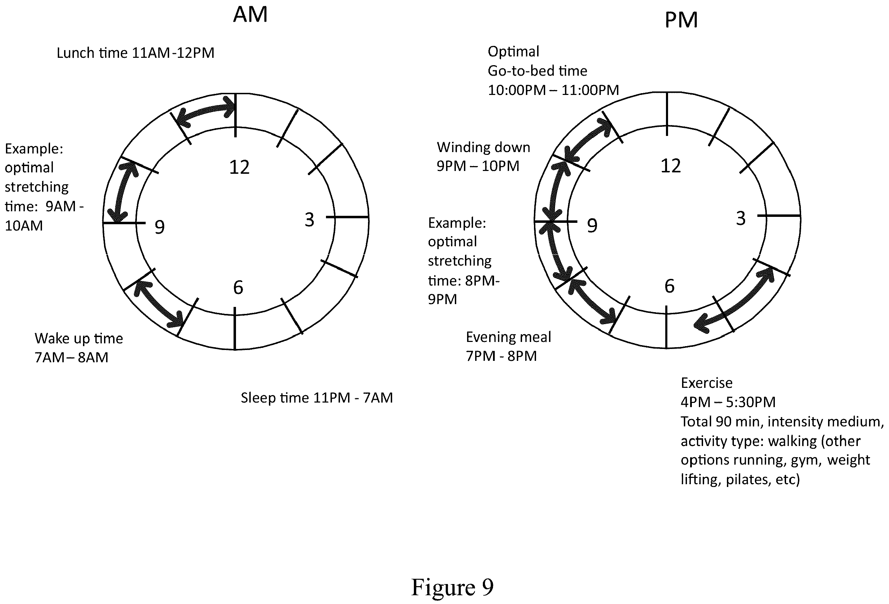

FIG. 9 shows examples, how to select an optimal stretching time in the user's daily schedule. In other words, this is for describing an optimal stretching time and input to select it (training, sleep time, go-to-bed time, wake-up time). If the exercise has been in the previous day in the late evening or earlier in the previous day, it might be that the stretching has not been done. Whether stretching has been done or not can be analyzed from the user activity measured by the wearable device as discussed above. The system may recommend guiding to do the stretching in the morning between 8-11 AM, and for example between 9-10 AM. If this was not possible to the user (such possibilities can be detected by a wearable device and by activity analysis), another optimal stretching time can be shown to be between 8-9 PM, for instance. This time is also optimal if the user has a training session in the morning or afternoon or early evening in the same day. If the user is not having an exercise this day, another optimal stretching time can be between 4-6 PM (i.e. in late afternoon).

In many embodiments, the system will guide to do stretching close to the go-to-bed time, typically 2-3 hours before that. This will help to relax at the same time and to give muscles optimal time to recover and also this helps to fall into sleep quickly. However, other times such as late afternoon or morning time can be proposed and scheduled depending on user's daily schedule.

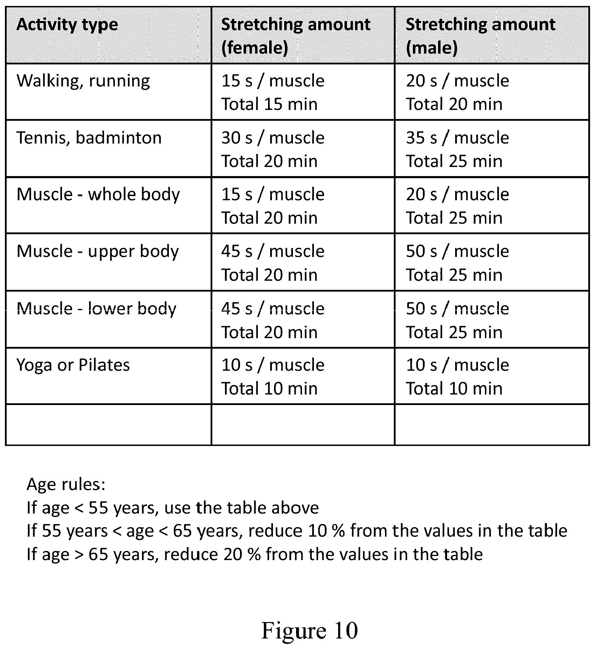

Different exercises load muscles in different ways. Stretching needs to vary according to the exercise done and also concerning the duration of the proposed stretching. A table in FIG. 10 gives some exemplary rules for stretching needs for different exercises. The stretching amount may be different to males and females, respectively. Furthermore, the age of a user can be taken into consideration as well.

In this exemplary table, the listed possible activity types are walking, running, tennis, badminton, whole body muscles, upper body muscles, lower body muscles, yoga and Pilates. Stretching amounts per muscle may vary between 10 seconds and 50 seconds there. The total stretching time may vary between 10 and 25 minutes there.

For example, after tennis exercise the stretching amount for a female user can be 30 seconds per muscle for a total of 20 minutes. A further instruction may be, for example, that muscles to be stretched are arms, wrists, back, shoulders, ham-strings, and legs. Each muscle will be stretched for 30 seconds and then kept in 10 seconds' rest, and then continued to the next muscle repeating until the total stretching time is full.

Age rule(s) can tune a general rule (in the actual table) to a more convenient one for an older user. An age rule can be that if the user is younger than 55 years, he/she is guided to use the stretching amounts in the table of FIG. 10. If the user is between 55-65 years, the stretching amount can be reduced 10 % from the table values, and if the user is older than 65 years, the stretching amount can be reduced 20 % from the table values. Of course, some other reduction percentages can be applied as well, or other age limits, too.

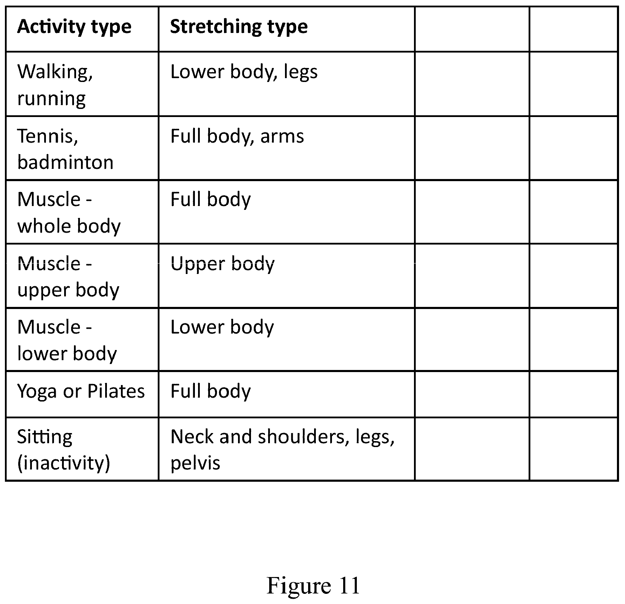

A table in FIG. 11 shows general guidelines for stretching different muscles after each exercise or activity type, in an embodiment. Different stretching books, programs and web-sources such as Youtube videos can be used to get more detailed and accurate muscle level instructions to each muscle or muscle group.

In this exemplary table, the listed possible activity types are walking, running, tennis, badminton, whole body muscles, upper body muscles, lower body muscles, yoga and Pilates and also, an inactivity type of sitting. The stretching types are listed in the table according to an embodiment, and the body parts to be stretched there are named among the lower body, legs, full body, arms, upper body, neck and shoulders, and pelvis.

In an embodiment, the server arrangement 108 is further operable to store the measured circadian rhythm, daily schedule, the measured duration of sleep, and activity type, activity amount, activity duration and activity time of the user 102, and stretching index of the user 102. For example, the measured circadian rhythm, activity type and the measured duration of sleep may be stored in a database of the server arrangement 108. In an embodiment, the operation and working of the server arrangement 108 and the database can be implemented with a dedicated computer system, a cluster of computers and/or a cloud service (or any combination thereof). Furthermore, the stored information combined to an input by the user 102 can be used in a step of re-calibration of the wearable electronic device 104. As mentioned above, the input by the user 102 may be associated with the external data, which comprises at least one of travel information, time zone, calendar, working schedule, and holidays.

In the present disclosure, the term "sleep score" may relate to a score provided to a sleep that the user may obtain in a span of a day (namely, a duration of 24 hours). Specifically, the sleep score of the sleep of the user may be based on at least one of: a time of falling asleep (namely, a go-to-bed time), wake-up time, circadian rhythm and physical parameters of the user, quality of the sleep (namely, sleep efficiency), sleep onset latency and so forth. In an example, the sleep score may be a numerical score or an alphabetic or an alphanumeric grade. Specifically, the numerical score may be determined on a scale of zero to 100. In such an example, a numerical score higher than 80 may be indicative of an optimum sleep duration. Similarly, in such an example, a numerical score lower than 70 may be indicative of a low sleep duration. Furthermore, the user 102 may obtain a high sleep score by sleeping an adequate number of hours at times coinciding with the circadian rhythm of the user 102.

In an embodiment, the server arrangement 108 is operable to measure the sleep efficiency of the user 102. Specifically, the sleep efficiency is indicative of a quality of sleep of the user 102. More specifically, the sleep efficiency may be based on factors such as movements of the user 102 while asleep (indicative of restlessness and wake-ups while in bed; and more deeper causes can be stress or use of alcohol in the previous day), duration of sleep in a deep sleep stage, rapid eye movement (i.e. REM) data, hypnogram and so forth. Furthermore, the sleep efficiency may be a numerical grade. Optionally, the server arrangement 108 is operable to determine the sleep efficiency based on the set of measurement data received from the wearable electronic device 104.

The server arrangement 108 is operable to analyze the sleep score to determine an optimum bedtime window for the user 102. Specifically, the server arrangement 108 is operable to determine a length of the optimum bedtime window, and a start time and an end time of the optimum bedtime window.

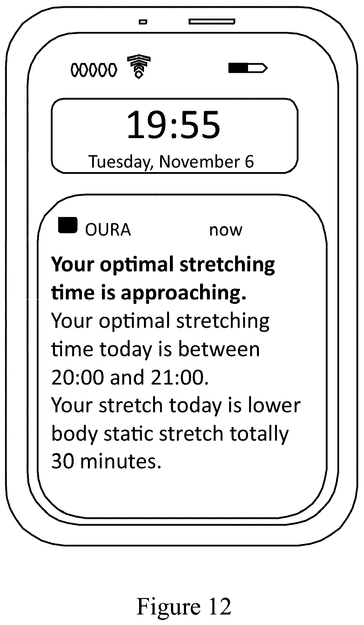

The system will generate a practice stretching guide and respective alert to the user 102. An example of the alert and stretching guide is shown in FIG. 12. The exemplary piece of information to the mobile device user may be as follows: "Your optimal stretching time is approaching. Your optimal stretching time today is between 20:00 and 21:00. Your stretch today is lower body static stretch totally 30 minutes." It is noted that 8 PM corresponds to 20:00 and 9 PM corresponds to 21:00. As it can be seen from the date and time information of the smart phone (or tablet), the weekday is Tuesday, and this alert is provided to the user five minutes before the optimal stretching time is due to start. The alert time can be specified also differently than 5 minutes before the optimal stretching time window is about to start.

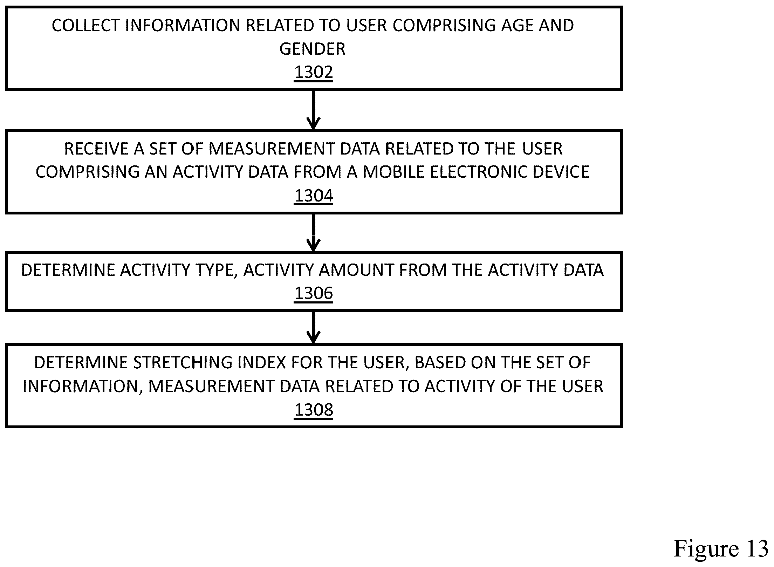

FIG. 13 shows process steps for determining stretching index for the user, in an embodiment. At first in this first process, the system collects information related to user comprising age and gender 1302. Secondly, the system receives a set of measurement data related to the user comprising activity data from a mobile electronic device 1304. Thirdly, the system determines an activity type, and activity amount from the activity data 1306. Finally, as a fourth step, the system determines a stretching index for the user based on the set of information, and measurement data related to activity of the user 1308.

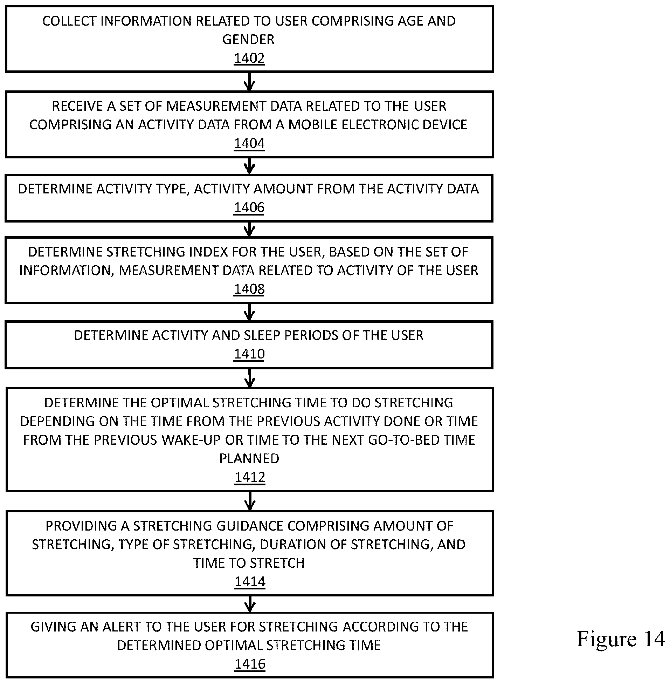

FIG. 14 shows process steps for giving an alert to the user for stretching. At first in this second process, the system collects information related to user comprising age and gender 1402. Secondly, the system receives a set of measurement data related to the user comprising activity data from a mobile electronic device 1404. Thirdly, the system determines an activity type, and activity amount from the activity data 1406. Fourthly, the system determines a stretching index for the user, based on the set of information, and measurement data related to the activity of the user 1408. Fifthly, the system determines activity and sleep periods of the user 1410. Sixthly, the system determines the optimal stretching time to do stretching depending on the time from the previous activity done or time from the previous wake-up or time to the next go-to-bed time planned 1412. Seventhly, the system provides stretching guidance comprising amount of stretching, type of stretching, duration of stretching, and a time to stretch 1414. Finally, as an eighth step, the system gives an alert to the user for stretching according to the determined optimal stretching time 1416.

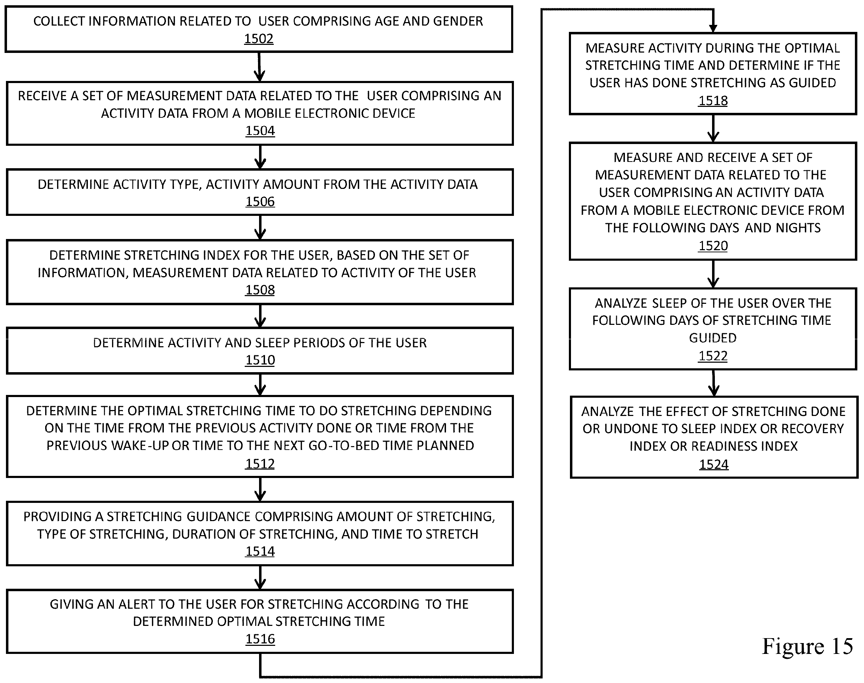

FIG. 15 shows process steps for analyzing the effects of the stretching done or undone to the user him/herself. At first in this third process, the system collects information related to user comprising age and gender 1502. Secondly, the system receives a set of measurement data related to the user comprising activity data from a mobile electronic device 1504. Thirdly, the system determines an activity type, and activity amount from the activity data 1506. Fourthly, the system determines a stretching index for the user, based on the set of information, and measurement data related to the activity of the user 1508. Fifthly, the system determines activity and sleep periods of the user 1510. Sixthly, the system determines the optimal stretching time to do stretching depending on the time from the previous activity done or time from the previous wake-up or time to the next go-to-bed time planned 1512. Seventhly, the system provides stretching guidance comprising amount of stretching, type of stretching, duration of stretching, and a time to stretch 1514. Eighthly, the system gives an alert to the user for stretching according to the determined optimal stretching time 1516. As the ninth step in the right-hand side of FIG. 15, the system measures activity during the optimal stretching time and determines if the user has done stretching as guided 1518. As the tenth step, the system measures and receives a set of measurement data related to the user comprising activity data from a mobile electronic device from the following days and nights 1520. As the eleventh step, the system analyzes sleep of the user over the following days of stretching time guided 1522. Finally, as the twelfth step, the system analyzes the effect of stretching done or undone to sleep index or recovery index or readiness index 1524.

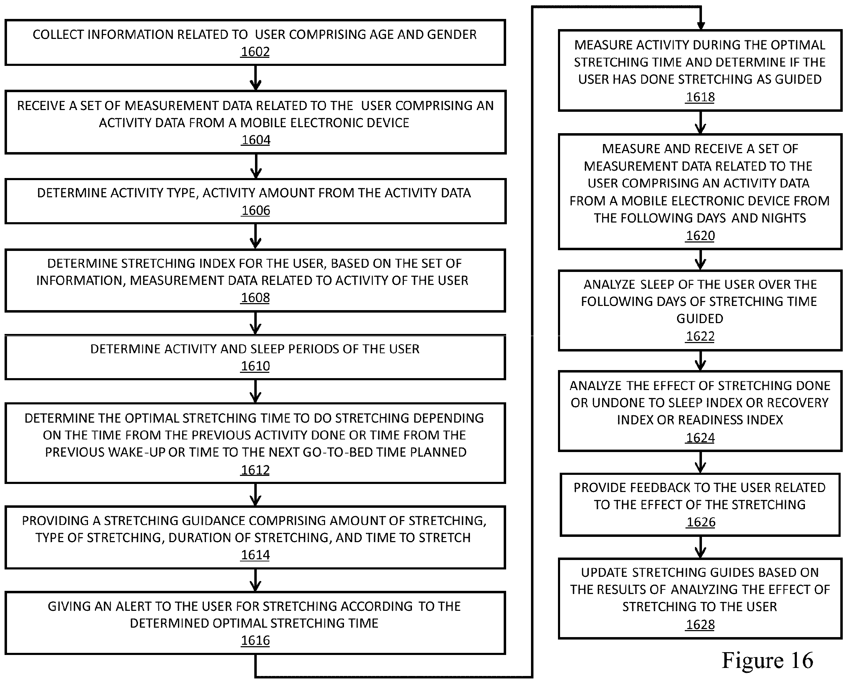

FIG. 16 shows process steps for providing feedback to the user related to stretching and sleep, and for updating stretching guides based on the analysis of the effect of stretching to the user. At first in this fourth process, the system collects information related to user comprising age and gender 1602. Secondly, the system receives a set of measurement data related to the user comprising activity data from a mobile electronic device 1604. Thirdly, the system determines an activity type, and activity amount from the activity data 1606. Fourthly, the system determines a stretching index for the user, based on the set of information, and measurement data related to the activity of the user 1608. Fifthly, the system determines activity and sleep periods of the user 1610. Sixthly, the system determines the optimal stretching time to do stretching depending on the time from the previous activity done or time from the previous wake-up or time to the next go-to-bed time planned 1612. Seventhly, the system provides stretching guidance comprising amount of stretching, type of stretching, duration of stretching, and a time to stretch 1614. Eighthly, the system gives an alert to the user for stretching according to the determined optimal stretching time 1616. As the ninth step in the right-hand side of FIG. 16, the system measures activity during the optimal stretching time and determines if the user has done stretching as guided 1618.

As the tenth step, the system measures and receives a set of measurement data related to the user comprising activity data from a mobile electronic device from the following days and nights 1620. As the eleventh step, the system analyzes sleep of the user over the following days of stretching time guided 1622. As the twelfth step, the system analyzes the effect of stretching done or undone to sleep index or recovery index or readiness index 1624. As the thirteenth step, the system provides feedback to the user related to the effect of the stretching 1626. Finally, as the fourteenth step, the system updates stretching guides based on the results of analyzing the effect of stretching to the user 1628.

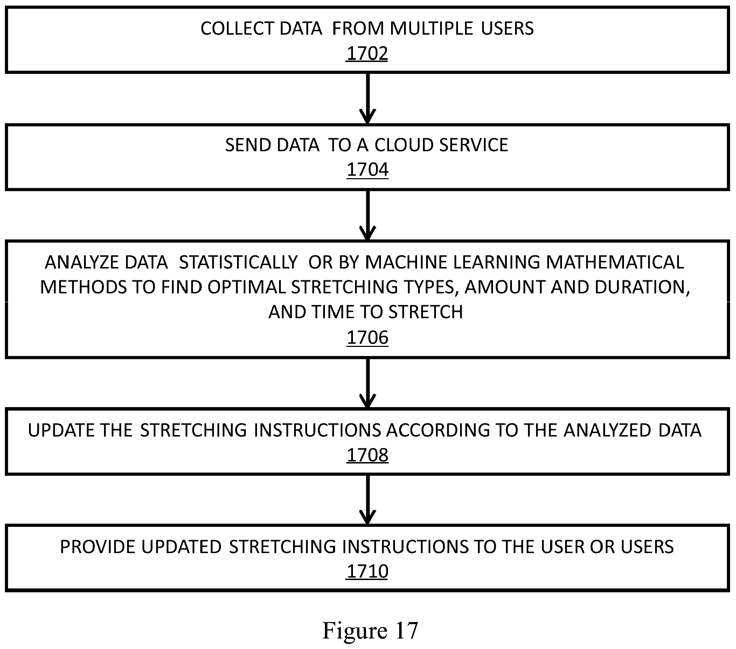

FIG. 17 shows process steps for benefiting from data gathered from multiple users. At first in this fifth process, the system collects data from multiple users 1702. Secondly, the system sends data to a cloud service 1704. Thirdly, the system analyzes data statistically or by machine learning mathematical methods to find optimal stretching types, amount and duration, and time to stretch 1706. Fourthly, the system updates the stretching instructions according to the analyzed data 1708. Finally, as the fifth step, the system provides updated stretching instructions to the user or users 1710. In other words, the fifth process is able to update stretching guidelines (instructions) based on one or more users' data related to the stretching done and the sleep scores.

The advantages of the present invention are that the presented processes and presented system for intelligent stretching guidance for users makes the recovery from an exercise quicker for professional and recreational sports exercisers. Also, the sleep quality may improve, and the results in the sports activities themselves may well enhance by the application of the present invention. General quality of life can get better with the present invention, because it allows for optimized work / free time balance, and also for optimized training / recovery time balance. The present invention thus enables the user to be fresher and less tired during the daytime as well, supporting an efficient worktime for normal taxpaying citizen and/or efficient training time for professional/recreational athletes.

The present invention is not restricted merely to the embodiments presented in the above but the present invention may vary within the scope of the claims.

## Claims

1. A method for providing optimal stretching guidance to a user (102) by analyzing physical activities of the user (102), wherein the method comprises the steps of:

   - collecting (1302-1602) a set of information related to the user (102) comprising an age and gender;
   - measuring and receiving (1304-1604) a set of measurement data related to the user (102) comprising activity data of the user (102) by a mobile communication device (106) or by a wearable electronic device (104);

- determining (1308-1608) a stretching index for the user (102), based on the set of information related to the user (102), and the set of measurement data related to the user (102), the stretching index basing at least on the activity data of the user (102);

- providing stretching guidance to the user (102) via the mobile communication device (106), where the stretching guidance is based on the stretching index for the user (102); **characterized in that** the method further comprises the step of

- providing feedback to the user (102) related to the effect of the stretching done, via the mobile communication device (106).

2.  The method according to claim 1, **characterized in that** the stretching index comprises at least one of the following: an optimal stretching type, an optimal amount or duration of stretching and an optimal stretching time of the day.

3.  The method according to any of claims 1 - 2, **characterized in that** the method further comprises the steps of:

    - determining activity and sleep periods of the user (102), and
    - determining the optimal stretching time depending on the time from a previous activity done or time from a previous wake-up or time to a next planned go-to-bed time.

4.  The method according to any of claims 1-3, **characterized in that** the method further comprises the step of:

    - providing stretching guidance to the user (102) comprising at least one of stretching type, amount or duration of stretching, and stretching time of the day, wherein the stretching guidance is based on the stretching index for the user (102).

5.  The method according to claim 4, **characterized in that** the method further comprises the steps of:

    - collecting data from multiple users;
    - sending collected data to a server (108) or to a cloud service;
    - analyzing the data statistically or by machine-learning mathematical methods to find optimal stretching types, optimal amounts or durations of stretching and optimal stretching times of the day;

    - updating at least one stretching guidance according to analysis results; and
    - providing the updated at least one stretching guidance to the user (102), in place of the stretching guidance of claim 4, with parameters of claim 4.

6.  The method according to claim 2, **characterized in that** the method further comprises the steps of:

    - measuring activity of the user (102) during the optimal stretching time; and
    - determining if the user (102) has done stretching as guided.

7.  The method according to any of claims 1-4 or claim 6, **characterized in that** the method further comprises the steps of:

    - measuring and receiving a set of measurement data related to the user (102) comprising activity data of the user (102) from the mobile communication device (106) in following days and nights;
    - analyzing sleep of the user (102) over the following 24-hour periods for a predetermined number of periods, when also stretching guidance is given to the user (102) during these periods; and
    - analyzing an effect of the performed stretching to a sleep index or a recovery index or a readiness index of the user (102).

8.  The method according to claim 1, **characterized in that** the method further comprises the step of:

    - updating stretching guidance based on the results of analyzing the effect of the performed stretching to the user (102).

9.  A system for providing optimal stretching guidance to a user (102) by analyzing physical activities of the user (102), wherein the system comprises:

    - a wearable electronic device (104);
    - a mobile communication device (106); and
    - a server (108);
    - the server (108) is configured to collect (1302-1602) a set of information related to the user (102) comprising an age and gender;
    - the wearable electronic device (104) is configured to measure and the server (108) is configured to receive (1304-1604) a set of measurement data related to the user (102) comprising activity data of the user (102) by a mobile communication device (106) or by a wearable electronic device (104);

- the server (108) is configured to determine (1308-1608) a stretching index for the user (102), based on the set of information related to the user (102), and the set of measurement data related to the user (102), the stretching index basing at least on the activity data of the user (102);

- the mobile communication device (106) is configured to provide stretching guidance to the user (102) via the mobile communication device (106), where the stretching guidance is based on the stretching index for the user (102); **characterized in that**

- the mobile communication device (106) is configured to provide feedback to the user (102) related to the effect of the stretching done.

10. The system according to claim 9, **characterized in that** the system further comprises the mobile communication device (106) which is configured to transmit the set of measurement data related to the user (102) to the server (108) for analysis, or the measurement and transmission are both configured to be performed by the wearable electronic device (104) and the mobile communication device (106) as a combined device.

11. The system according to claim 9, **characterized in that** the wearable electronic device (104) comprises at least one of the following: a heart rate sensor, a light sensor, an activity sensor, a temperature sensor, a rechargeable battery, an optional sensor, a microprocessor (MCU), a memory, an output indicator comprising a piezo and/or LED indicator, and a communication unit comprising wireless and/or Bluetooth transmission.

12. The system according to claim 9, **characterized in that** the mobile communication device (106) comprises at least one of the following: an input device comprising at least one of a touchpad, a touch display, a microphone, a camera and a battery; an output device comprising at least one of a display, a piezo element and a speaker; a rechargeable battery; an optional sensor comprising at least one of a light, location, GPS, and motion sensor; a microprocessor (MCU); a memory; a wireless communication unit to the wearable electronic device (104); and a wireless communication unit to a network (110).

13. The system according to claim 9, **characterized in that** a network (110) comprises at least one of the following: a microprocessor (MCU); a memory; an output indicator comprising a piezo and/or LED indicator; a wireless communication unit to the mobile communication device (106); and a wireless or wired communication unit to the server (108).

14. The system according to claim 9, **characterized in that** the server (108) comprises at least one of the following: an input device comprising at least one of a touchpad, a touch display, a microphone, a camera and a battery; an output device comprising at least one of a display, a piezo element and a speaker; a power unit; a microprocessor (MCU); a memory; a wireless or wired communication unit to the network (110); and a database.

15. A computer program product for providing optimal stretching guidance to a user (102) by analyzing physical activities of the user (102), wherein the computer program product comprises program code, which is executable when run in a processor, wherein the computer program product is configured to execute the steps of:

- collecting (1302-1602) a set of information related to the user (102) comprising an age and gender;

- measuring and receiving (1304-1604) a set of measurement data related to the user (102) comprising activity data of the user (102) by a mobile communication device (106) or by a wearable electronic device (104);

- determining (1308-1608) a stretching index for the user (102), based on the set of information related to the user (102), and the set of measurement data related to the user (102), the stretching index basing at least on the activity data of the user (102);

- providing stretching guidance to the user (102) via the mobile communication device (106), where the stretching guidance is based on the stretching index for the user (102); **characterized in that** the computer program product is further configured to execute the step of

- providing feedback to the user (102) related to the effect of the stretching done, via the mobile communication device (106).

Figure 1

## AM

Lunch time 11 AM – 12 PM

Morning
work
session
8.30 – 11
AM

Wake-up time
7 AM – 8 AM

Sleep time 11 PM – 7 AM

## PM

Optimal
go-to-bed time
10:00 PM – 11:00 PM

Afternoon
work
session
12 AM – 4
PM

Winding down
9 PM – 10 PM

Evening meal
7 PM – 8 PM

Exercise
4 PM – 5:30 PM

Figure 2

EP 3 855 447 A1

## WEARABLE ELECTRONIC DEVICE
### 104

| | |
|---|---|
| Heart rate sensor | Light sensor |
| Activity sensor | Temperature sensor |
| Rechargeable battery | Optional sensor |
| Microprocessor (MCU) | Memory |
| Output indicator (piezo, LED) | Communication unit (wireless, BT) |

Figure 3

## MOBILE COMMUNICATION DEVICE
### 106

| | |
|---|---|
| Input device (touchpad, touch display, microphone, camera, battery) | Output device (display, piezo, speaker) |
| Rechargeable battery | Optional sensor (light, location, GPS, motion) |
| Microprocessor (MCU) | Memory |
| Communication unit A (wireless) to the wearable device | Communication unit B (wireless) to a network |

Figure 4

EP 3 855 447 A1

## NETWORK
## 110

Microprocessor (MCU)

Memory

Output indicator (piezo, LED)

Communication unit C (wireless)

Communication unit D (wireless or wired)

Figure 5

## SERVER
## 108

Input device (touchpad, touch display, microphone, camera, battery)

Output device (display, piezo, speaker)

Power unit

Microprocessor (MCU)

Memory

Communication unit E (wireless or wired)

Database

Figure 6

EP 3 855 447 A1

Figure 7

EP 3 855 447 A1

Figure 8

EP 3 855 447 A1

AM

PM

Lunch time 11AM-12PM

Optimal
Go-to-bed time
10:00PM – 11:00PM

Example:
optimal
stretching
time: 9AM -
10AM

Winding down
9PM – 10PM

12

12

3

3

9

9

Example:
optimal
stretching
time: 8PM-
9PM

6

6

Wake up time
7AM – 8AM

Evening meal
7PM - 8PM

Sleep time 11PM - 7AM

Exercise
4PM – 5:30PM
Total 90 min, intensity medium,
activity type: walking (other
options running, gym, weight
lifting, pilates, etc)

EP 3 855 447 A1

Figure 9

| Activity type | Stretching amount (female) | Stretching amount (male) |
|---|---|---|
| Walking, running | 15 s / muscle Total 15 min | 20 s / muscle Total 20 min |
| Tennis, badminton | 30 s / muscle Total 20 min | 35 s / muscle Total 25 min |
| Muscle - whole body | 15 s / muscle Total 20 min | 20 s / muscle Total 25 min |
| Muscle - upper body | 45 s / muscle Total 20 min | 50 s / muscle Total 25 min |
| Muscle - lower body | 45 s / muscle Total 20 min | 50 s / muscle Total 25 min |
| Yoga or Pilates | 10 s / muscle Total 10 min | 10 s / muscle Total 10 min |
| | | |

Age rules:

If age < 55 years, use the table above

If 55 years < age < 65 years, reduce 10 % from the values in the table

If age > 65 years, reduce 20 % from the values in the table

Figure 10

| Activity type | Stretching type | | |
|---|---|---|---|
| Walking, running | Lower body, legs | | |
| Tennis, badminton | Full body, arms | | |
| Muscle - whole body | Full body | | |
| Muscle - upper body | Upper body | | |
| Muscle - lower body | Lower body | | |
| Yoga or Pilates | Full body | | |
| Sitting (inactivity) | Neck and shoulders, legs, pelvis | | |

Figure 11

EP 3 855 447 A1

00000 📶 ▬▭

**19:55**

Tuesday, November 6

■ OURA          now

**Your optimal stretching time is approaching.**
Your optimal stretching time today is between 20:00 and 21:00.
Your stretch today is lower body static stretch totally 30 minutes.

Figure 12

```
┌─────────────────────────────────────────────────────────────────┐
│        COLLECT INFORMATION RELATED TO USER COMPRISING AGE AND     │
│                             GENDER                                │
│                              1302                                 │
└─────────────────────────────────────────────────────────────────┘
                                │
                                ▼
┌─────────────────────────────────────────────────────────────────┐
│         RECEIVE A SET OF MEASUREMENT DATA RELATED TO THE USER     │
│     COMPRISING AN ACTIVITY DATA FROM A MOBILE ELECTRONIC DEVICE   │
│                              1304                                 │
└─────────────────────────────────────────────────────────────────┘
                                │
                                ▼
┌─────────────────────────────────────────────────────────────────┐
│    DETERMINE ACTIVITY TYPE, ACTIVITY AMOUNT FROM THE ACTIVITY DATA │
│                              1306                                 │
└─────────────────────────────────────────────────────────────────┘
                                │
                                ▼
┌─────────────────────────────────────────────────────────────────┐
│      DETERMINE STRETCHING INDEX FOR THE USER, BASED ON THE SET OF │
│     INFORMATION, MEASUREMENT DATA RELATED TO ACTIVITY OF THE USER │
│                              1308                                 │
└─────────────────────────────────────────────────────────────────┘
```

Figure 13

```
┌─────────────────────────────────────────────────────────┐
│      COLLECT INFORMATION RELATED TO USER COMPRISING AGE AND │
│                        GENDER                              │
│                         1402                               │
└─────────────────────────────────────────────────────────┘
                            ↓
┌─────────────────────────────────────────────────────────┐
│      RECEIVE A SET OF MEASUREMENT DATA RELATED TO THE USER  │
│   COMPRISING AN ACTIVITY DATA FROM A MOBILE ELECTRONIC DEVICE │
│                         1404                               │
└─────────────────────────────────────────────────────────┘
                            ↓
┌─────────────────────────────────────────────────────────┐
│  DETERMINE ACTIVITY TYPE, ACTIVITY AMOUNT FROM THE ACTIVITY DATA │
│                         1406                               │
└─────────────────────────────────────────────────────────┘
                            ↓
┌─────────────────────────────────────────────────────────┐
│    DETERMINE STRETCHING INDEX FOR THE USER, BASED ON THE SET OF │
│  INFORMATION, MEASUREMENT DATA RELATED TO ACTIVITY OF THE USER │
│                         1408                               │
└─────────────────────────────────────────────────────────┘
                            ↓
┌─────────────────────────────────────────────────────────┐
│       DETERMINE ACTIVITY AND SLEEP PERIODS OF THE USER     │
│                         1410                               │
└─────────────────────────────────────────────────────────┘
                            ↓
┌─────────────────────────────────────────────────────────┐
│   DETERMINE THE OPTIMAL STRETCHING TIME TO DO STRETCHING   │
│  DEPENDING ON THE TIME FROM THE PREVIOUS ACTIVITY DONE OR TIME │
│  FROM THE PREVIOUS WAKE-UP OR TIME TO THE NEXT GO-TO-BED TIME │
│                        PLANNED                             │
│                         1412                               │
└─────────────────────────────────────────────────────────┘
                            ↓
┌─────────────────────────────────────────────────────────┐
│     PROVIDING A STRETCHING GUIDANCE COMPRISING AMOUNT OF   │
│   STRETCHING, TYPE OF STRETCHING, DURATION OF STRETCHING, AND │
│                     TIME TO STRETCH                        │
│                         1414                               │
└─────────────────────────────────────────────────────────┘
                            ↓
┌─────────────────────────────────────────────────────────┐
│  GIVING AN ALERT TO THE USER FOR STRETCHING ACCORDING TO THE │
│           DETERMINED OPTIMAL STRETCHING TIME               │
│                         1416                               │
└─────────────────────────────────────────────────────────┘
```

Figure 14

COLLECT INFORMATION RELATED TO USER COMPRISING AGE AND GENDER
1502

RECEIVE A SET OF MEASUREMENT DATA RELATED TO THE USER COMPRISING AN ACTIVITY DATA FROM A MOBILE ELECTRONIC DEVICE
1504

DETERMINE ACTIVITY TYPE, ACTIVITY AMOUNT FROM THE ACTIVITY DATA
1506

DETERMINE STRETCHING INDEX FOR THE USER, BASED ON THE SET OF INFORMATION, MEASUREMENT DATA RELATED TO ACTIVITY OF THE USER
1508

DETERMINE ACTIVITY AND SLEEP PERIODS OF THE USER
1510

DETERMINE THE OPTIMAL STRETCHING TIME TO DO STRETCHING DEPENDING ON THE TIME FROM THE PREVIOUS ACTIVITY DONE OR TIME FROM THE PREVIOUS WAKE-UP OR TIME TO THE NEXT GO-TO-BED TIME PLANNED
1512

PROVIDING A STRETCHING GUIDANCE COMPRISING AMOUNT OF STRETCHING, TYPE OF STRETCHING, DURATION OF STRETCHING, AND TIME TO STRETCH
1514

GIVING AN ALERT TO THE USER FOR STRETCHING ACCORDING TO THE DETERMINED OPTIMAL STRETCHING TIME
1516

MEASURE ACTIVITY DURING THE OPTIMAL STRETCHING TIME AND DETERMINE IF THE USER HAS DONE STRETCHING AS GUIDED
1518

MEASURE AND RECEIVE A SET OF MEASUREMENT DATA RELATED TO THE USER COMPRISING AN ACTIVITY DATA FROM A MOBILE ELECTRONIC DEVICE FROM THE FOLLOWING DAYS AND NIGHTS
1520

ANALYZE SLEEP OF THE USER OVER THE FOLLOWING DAYS OF STRETCHING TIME GUIDED
1522

ANALYZE THE EFFECT OF STRETCHING DONE OR UNDONE TO SLEEP INDEX OR RECOVERY INDEX OR READINESS INDEX
1524

Figure 15

Figure 16

COLLECT DATA FROM MULTIPLE USERS
1702

SEND DATA TO A CLOUD SERVICE
1704

ANALYZE DATA STATISTICALLY OR BY MACHINE LEARNING MATHEMATICAL METHODS TO FIND OPTIMAL STRETCHING TYPES, AMOUNT AND DURATION, AND TIME TO STRETCH
1706

UPDATE THE STRETCHING INSTRUCTIONS ACCORDING TO THE ANALYZED DATA
1708

PROVIDE UPDATED STRETCHING INSTRUCTIONS TO THE USER OR USERS
1710

Figure 17

Europäisches
Patentamt

European
Patent Office

Office européen
des brevets

# EUROPEAN SEARCH REPORT

**Application Number**

EP 21 15 2971

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2019/366154 A1 (CALLAGHAN DAVID M [US]) 5 December 2019 (2019-12-05) * paragraphs [0039], [0014], [0053], [0039], [0020], [0031], [0018], [0033], [0063]; figures 1,3 * ----- | 1-15 | INV.<br>G16H20/30<br>G16H40/63<br>G16H40/67<br>G16H50/20<br>G16H50/70 |
| | | | **TECHNICAL FIELDS SEARCHED (IPC)**<br><br>G16H |

The present search report has been drawn up for all claims

| Place of search | Date of completion of the search | Examiner |
|---|---|---|
| Munich | 12 May 2021 | Samulowitz, Michael |

CATEGORY OF CITED DOCUMENTS

X : particularly relevant if taken alone
Y : particularly relevant if combined with another
  document of the same category
A : technological background
O : non-written disclosure
P : intermediate document

T : theory or principle underlying the invention
E : earlier patent document, but published on, or
  after the filing date
D : document cited in the application
L : document cited for other reasons

& : member of the same patent family, corresponding
  document

EPO FORM 1503 03.82 (P04C01)

## EP 3 855 447 A1

**ANNEX TO THE EUROPEAN SEARCH REPORT
ON EUROPEAN PATENT APPLICATION NO.**

EP 21 15 2971

12-05-2021

| Patent document cited in search report | Publication date | Patent family member(s) | Publication date |
|---|---|---|---|
| US 2019366154 A1 | 05-12-2019 | US 2019366154 A1<br>WO 2019231729 A1 | 05-12-2019<br>05-12-2019 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20110184247 A
- US 20170206795 A